# DEMANDE DE BREVET EUROPEEN

(11) **EP 0 612 505 A1**
(43) Date de publication de la demande: **31.08.1994**
(21) Numéro de dépôt: 93460004.0
(22) Date de dépôt: 25.01.1993
(51) Int. Cl.: A61B 17/12

(54) **Pince chirurgicale pour poser des clips de suture**

(71) Demandeur: SOCIETE VITACORP, F-35510 Cesson-Sévigné (FR)
(72) Inventeur: Bourles, Philippe, F-35136 Saint-Jacques-de-la-Lande (FR)
(74) Mandataire: Le Faou, Daniel

(57) **Abrégé**

Cette pince possède un magasin (7) dans lequel sont stockés et guidés les clips de suture en forme de "U", ces clips étant stockés et guidés dans un magasin linéaire (7) et poussés par un ressort en direction de l'extrémité ouverte (72) du magasin ; la pince est équipée d'un coulisseau (2) mobile en translation axiale au-dessus du magasin ; ce coulisseau est muni de moyens de commande permettant la fermeture d'une paire de mâchoires (30) disposées à la sortie du magasin ; le coulisseau (2) est également solidaire d'une languette de retenue (81) susceptible de s'escamoter momentanément vers le haut lors de l'avance du coulisseau pour permettre le transfert du premier clip (6b) de la rangée entre les mâchoires (30).

Matériel chirurgical pour la suture de vaisseaux sanguins.

## Description

La présente invention concerne une pince chirurgicale pour poser des clips de suture et plus précisément des clips ayant la forme générale d'un "U" que l'on sertit, par resserrement du clip, sur des vaisseaux sanguins.

Des instruments chirurgicaux de ce type sont bien connus. Ils contiennent une réserve de clips, par exemple de quinze ou vingt clips. Ils possèdent des organes de manoeuvre du genre branches de ciseaux. A l'aide de ces branches, le chirurgien peut d'une seule main poser le clip, les mécanismes internes à l'instrument assurant la distribution d'un nouveau clip de la réserve au cours de la manoeuvre.

Ce genre d'instrument, bien entendu aseptisé, est généralement utilisé une seule fois. Il est jeté à l'issue de l'intervention chirurgicale, que la réserve de clips soit ou non épuisée.

Ces pinces chirurgicales ne donnent pas entièrement satisfaction dans la mesure où elles comportent un grand nombre de pièces en mouvement, ce qui compromet sa fiabilité et est source de disfonctionnements. Or, on comprend aisément qu'au cours d'une intervention chirurgicale, une distribution sans faille soit essentielle. De plus, la complexité des mécanismes rend très onéreux le prix de revient de ce genre d'instruments, ce qui est d'autant plus fâcheux que l'instrument est jeté après une seule utilisation.

Comme illustration de l'état de la technique en la matière, on peut citer notamment les documents EP-B-170 592, EP-B-144 144, EP-A-324 549 et FR-A-2 637 489.

L'instrument qui fait l'objet de EP-B-170 592 comporte des moyens supportant une rangée de clips, ainsi qu'un moyen de fermeture de clips qui est mobile de l'avant vers l'arrière. Ce moyen porte une paire de mors qui, par suite de leur déplacement en direction distale, se trouvent rapprochés l'un de l'autre par des surfaces de came stationnaires. L'instrument comporte un dispositif d'échappement agencé pour placer le dernier clip de la rangée entre les mors, lorsque le moyen de fermeture mobile se trouve en position reculée, et pour retenir les autres clips. Un tel instrument, outre sa complexité mécanique, ne donne pas réellement satisfaction en raison du déplacement des mors de serrage au cours de la suture, ce qui est gênant car il provoque le déplacement du vaisseau à ligaturer.

L'instrument selon EP-B-144 144 comporte une paire de mâchoires mobiles dont l'actionnement est réalisé par des moyens du genre branches de ciseau. L'instrument comporte un moyen formant poussoir agencé pour transférer les clips un à un à la fois dans les mâchoires, depuis des moyens de stockage recevant les clips les uns derrière les autres. Il est prévu à l'intérieur des mâchoires un moyen formant butée qui autorise le transfert du clip vers l'avant, mais interdit son recul.

Ce dispositif est relativement complexe et coûteux.

Un dispositif similaire est décrit dans EP-A-324 549. L'instrument qui fait l'objet de ce document possède une paire de mâchoires actionnées par des moyens du type ciseaux, ces moyens étant également adaptés pour déplacer une barre pousseuse. La barre pousseuse, qui porte un appendice, est déplaçable d'une position initiale avancée dans laquelle l'appendice se trouve derrière le clip, entre les mâchoires, et une position reculée dans laquelle elle se trouve derrière le clip le plus en avant de la série, pour l'amener ensuite dans sa position avancée.

Ici aussi on a affaire à un mécanisme extrêmement complexe et source de disfonctionnements.

Le dispositif selon FR-A-2 637 489 est également fort complexe, dans la mesure où cet instrument comprend deux parties mobiles en translation et en sens inverse ; l'une des parties est le magasin renfermant la réserve de clips. L'autre partie est un fourreau assurant la fermeture des mâchoires. Les branches de manoeuvre, du genre ciseaux, entraînent deux jeux de biellettes, chacun de ces jeux commandant le déplacement de l'une des deux parties.

La présente invention vise à résoudre ces difficultés en proposant un instrument du type évoqué plus haut qui soit d'un fonctionnement fiable, d'un maniement simple, et d'un prix de revient bas.

L'objet de l'invention, qui sert à poser des clips de suture sur des vaisseaux sanguins, ces clips ayant la forme générale d'un "U", est du type comprenant un corps fixe sur lequel sont articulés deux leviers de manoeuvre, du genre branche de ciseaux, ce corps fixe contenant un magasin linéaire présentant une extrémité ouverte et renfermant une réserve de clips disposés à plat les uns contre les autres, leur ouverture tournée vers l'extrémité ouverte, la pince comportant une paire de mâchoires situées dans le prolongement du magasin, juste à la sortie de son extrémité ouverte, ces mâchoires étant adaptées pour retenir et pour sertir l'un après l'autre des clips provenant du magasin.

Ce type de pince fait partie de l'état de la technique.

Conformément à l'invention, la pince comporte un coulisseau disposé longitudinalement au-dessus du magasin et dont le mouvement de translation longitudinale (c'est-à-dire parallèlement à la direction longitudinale du magasin) est commandé par les leviers de manoeuvre, la fermeture de ceux-ci provoquant le déplacement du coulisseau dans le sens opposé à l'extrémité ouverte - sens qui dans la suite sera appelé "recul" - tandis que leur ouverture provoque le déplacement du coulisseau en sens inverse - sens qui sera appelé "avance" - ; la pince comporte également un ressort de poussée qui tend constamment à déplacer la réserve de clips vers l'extrémité ouverte du magasin, tandis que le coulisseau est solidaire, d'une part de moyens assurant la fermeture des mâchoires au cours de son recul, d'autre part d'une languette de retenue du premier clip contenu (côté extrémité ouverte) dans le magasin, des moyens assurant l'escamotage momentané de cette languette au cours de l'avance du coulisseau.

Par ailleurs, selon un certain nombre de caractéristiques avantageuses, mais non limitatives de l'invention :
- les mâchoires sont plates et contenues dans un plan général qui est légèrement incliné vers bas par rapport au plan du magasin ; cette disposition améliore la visibilité pour l'opérateur ;
- les mâchoires sont les extrémités des branches formées dans une même pièce (tige plate) et séparées par une fente, la fermeture des mâchoires se faisant sous déformation élastique de ces branches ;
- les moyens portés par le coulisseau, qui assurent la fermeture des mâchoires, consistent en un étrier en forme de "U" renversé dont les branches latérales s'appliquent contre des surfaces de came formées sur les bords extérieurs des branches des mâchoires ;
- les mâchoires sont munies de butées d'arrêt et de positionnement des clips ;
- la languette de retenue est constituée par l'extrémité recourbée d'une lame flexible fixée au coulisseau, et dont la précontrainte tend constamment à repousser la languette vers le bas, à l'intérieur du magasin, en position d'interception des clips ;
- les moyens qui assurent l'escamotage de la languette de retenue comprennent une rampe inclinée solidaire de la languette et une partie fixe formant contre-rampe ;
- la coopération de la rampe avec cette partie fixe, au cours de l'avance du coulisseau, provoque le soulèvement momentané de la languette de retenue, qui libère par conséquent le clip auquel elle était accrochée ;
- lorsque la languette de retenue est formée par l'extrémité d'une lame flexible, ladite rampe inclinée est formée de deux pattes découpées et repliées dans cette lame, tandis que la partie fixe formant contre-rampe est constituée par des languettes formant les bords d'une ouverture recevant la lame, ouverture ménagée dans une plaque ;
- cette plaque possède un bord présentant une échancrure en "V" disposée de manière à surplomber les mâchoires et à servir de butée et de centrage au vaisseau que l'on doit suturer ;
- la transmission du mouvement des leviers de manoeuvre au coulisseau est réalisée au moyen d'un jeu de biellettes.

D'autres caractéristiques et avantages de l'invention apparaîtront de la description et des dessins annexés qui en présentent un mode de réalisation préférentiel.

Sur ces dessins :
- la figure 1 est une vue de dessus schématique, avec arrachements partiels, d'une pince conforme à l'invention, avant une opération de suture d'un vaisseau sanguin ;
- la figure 2 est une vue analogue à la figure 1 montrant la pince à la fin de l'opération ;
- la figure 3 est une vue en perspective d'un clip ;
- la figure 4 montre en perspective l'extrémité du coulisseau qui porte les moyens de fermeture des mâchoires ;
- les figures 5 et 6 sont deux vues similaires qui représentent, en coupe longitudinale, l'extrémité de la pince, côté mâchoire, le coulisseau étant respectivement en position moyennement avancée et en position reculée ;
- la figure 7 est une vue en perspective, avec arrachement partiel, représentant la lame flexible qui porte la languette de retenue des clips, ainsi que la plaque fixe formant contre-rampe ;
- la figure 8 est une vue de dessus schématique au niveau de l'extrémité ouverte du magasin qui montre le transfert des clips dans les mâchoires ;
- les figures 9, 10 et 11 sont des vues de dessus schématiques du magasin et des mâchoires, ces vues illustrant trois étapes successives de mise en oeuvre de la pince en cours d'utilisation.

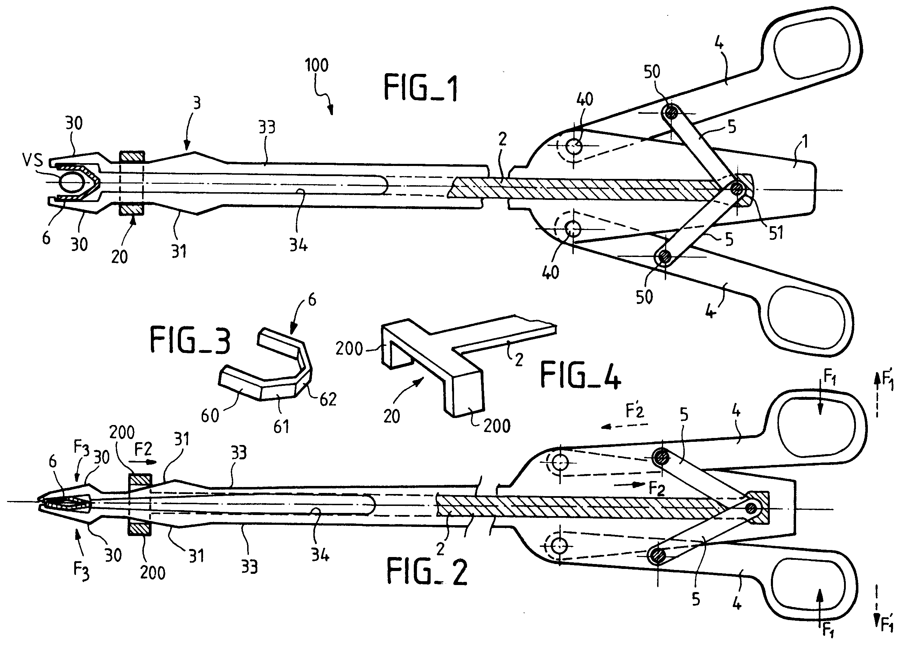

La pince chirurgicale 100 représentée à la figure 1 - disposée à plat, horizontalement - possède un corps fixe 1 ayant une forme allongée ; seule son extrémité arrière (partie droite de la figure) a été représentée sur le dessin.

Sur le corps 1 sont articulés, autour d'axes verticaux 40, une paire de leviers de manoeuvre 4 du genre branches de ciseaux, dont les extrémités libres sont conformées en anneaux permettant le passage des doigts. Sur chaque levier 4 est articulé, autour d'un axe vertical 50, une biellette 5. Les deux biellettes sont articulées, à leur extrémité opposée, autour d'un axe 51, sur l'extrémité d'un coulisseau 2. Celui-ci a la forme d'une tige, qui s'étend longitudinalement, coaxialement au corps fixe 1, et est guidé en translation dans celui-ci par des organes de guidage appropriés (glissières) non représentés.

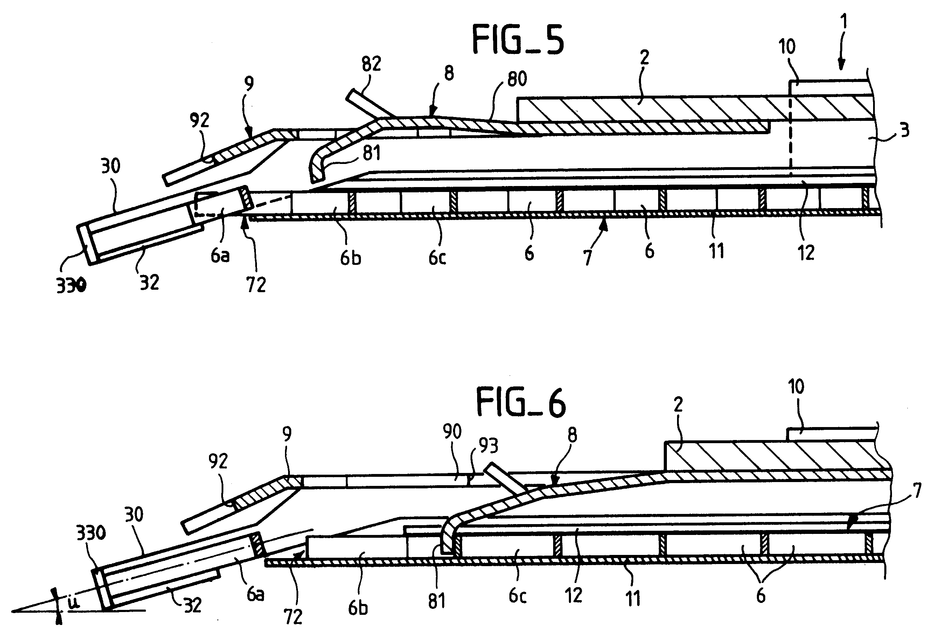

A son autre extrémité (partie gauche de la figure) le coulisseau 2 a la forme d'un étrier 20 en forme de "U" renversé, comme cela est visible à la figure 4. On a désigné par les références 200 les branches latérales verticales de l'étrier 20.

Dans le corps 1 est également montée une tige plate 3. Celle-ci est fixe, et présente une fente longitudinale 34 relativement longue, qui débouche à l'extrémité de la tige, du côté opposé aux leviers de manoeuvre 4 (à gauche sur la figure).

La fente 34 assure la séparation entre deux branches 33, dont les extrémités libres 30 ont la forme de mâchoires. La pièce 3 est réalisée dans un matériau, métallique ou plastique, ayant une bonne élasticité, de sorte qu'en appliquant sur les deux branches 33 un effort dirigé vers l'intérieur, on peut rapprocher l'une de l'autre les mâchoires 30. Sur les côtés des branches 33 sont prévues des pans inclinés 31 formant cames, aptes à coopérer avec les branches 200 de l'étrier 20, de telle sorte que le recul (déplacement vers la droite) de cet étrier provoque le rapprochement des deux mâchoires 30. Inversement, si l'étrier se déplace dans l'autre sens (avance) les branches 33 reviennent élastiquement à leur état initial représenté à la figure 1.

Les mâchoires 30 sont conformées pour recevoir entre elles un clip 6 de type connu. Celui-ci a la forme générale d'un "U". Ainsi, dans l'exemple représenté à la figure 3, le clip 6 comprend deux branches latérales 60 qui sont reliées à une portion transversale 62 (fond du "U") par des parties obliques 61. Le clip est formé dans un fil métallique de section rectangulaire.

Bien entendu, d'autres variantes de clips, ayant dans les détails une forme différente peuvent être utilisées, pourvu qu'ils aient une forme générale en "U".

A la figure 1, on a représenté un clip 6 en place entre les mâchoires 30. Au cours d'une intervention chirurgicale visant à ligaturer au moyen du clip un vaisseau sanguin VS, le chirurgien positionne la pince de telle manière que le clip 6 vienne entourer le vaisseau VS. Il agit ensuite sur les leviers 4 dans le sens de leur rapprochement (flèches F1, figure 2). Ce mouvement provoque, par l'intermédiaire des biellettes 5, un mouvement de translation longitudinale du coulisseau 2 dans le sens de son recul (flèche F2). L'étrier 20 provoque alors, en se déplaçant le long des cames 31, la fermeture des mâchoires 30 (flèches F3). Les mâchoires provoquent donc le sertissage du clip sur le vaisseau VS qui se trouve ainsi ligaturé.

Comme on le voit sur les figures 5 et 6, la partie fixe 1 possède une paroi inférieure plane 11 qui sert de fond de magasin pour la réserve de clips. On a désigné par la référence 10 la paroi supérieure de la partie fixe 1. Cette partie a la forme générale d'un fourreau recevant le coulisseau 2 et la pièce 3. Ce fourreau a une section générale rectangulaire, dont les parois sont échancrées à son extrémité avant afin de permettre le passage de l'étrier 20 et son accès aux branches 33 des mâchoires.

Les clips 6 sont stockés à plat les uns contre les autres sur le fond 11. Des guides latéraux 12 sous forme de nervures formés dans le corps 1 assurent la retenue des clips vers le haut. La réserve des clips est donc retenue et guidée dans un logement généralement tubulaire, appelé magasin linéaire 7, contenu dans le corps 1.

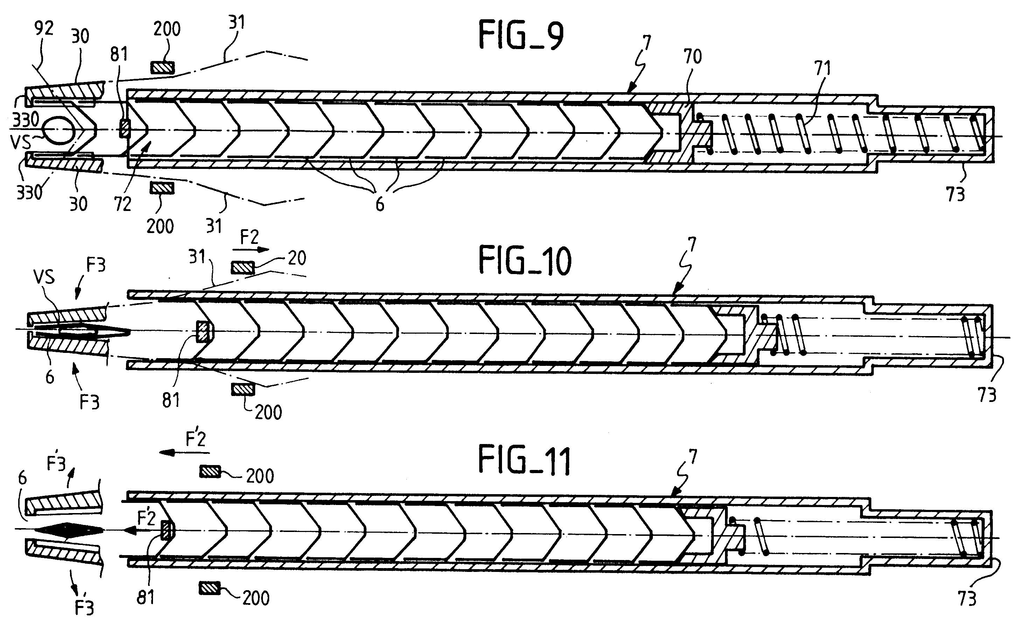

Comme on le voit aux figures 9 à 11, le magasin 7 présente une extrémité ouverte 72 qui débouche du côté des mâchoires 30 et une extrémité fermée 73. Celle-ci sert d'appui à un (ou plusieurs) ressort(s) de poussée 71 qui agi(ssen)t sur un piston 70 qui s'applique contre le dernier clip de la rangée. Le ressort 71 tend donc à repousser toute la réserve de clips vers son extrémité ouverte 72.

Sous le coulisseau 2 est fixé, par exemple par une soudure 85, une lame flexible 8. Celle-ci est recourbée vers le bas pour présenter une languette 81 à peu près perpendiculaire au plan général des clips, c'est-à-dire disposée verticalement si on considère que l'instrument se trouve placé horizontalement, ainsi que cela est illustré sur les dessins.

La languette 81 a une largeur qui correspond approximativement à celle du tronçon transversal 62 des clips, de sorte qu'elle puisse venir se placer à l'intérieur d'un clip, contre cette partie transversale.

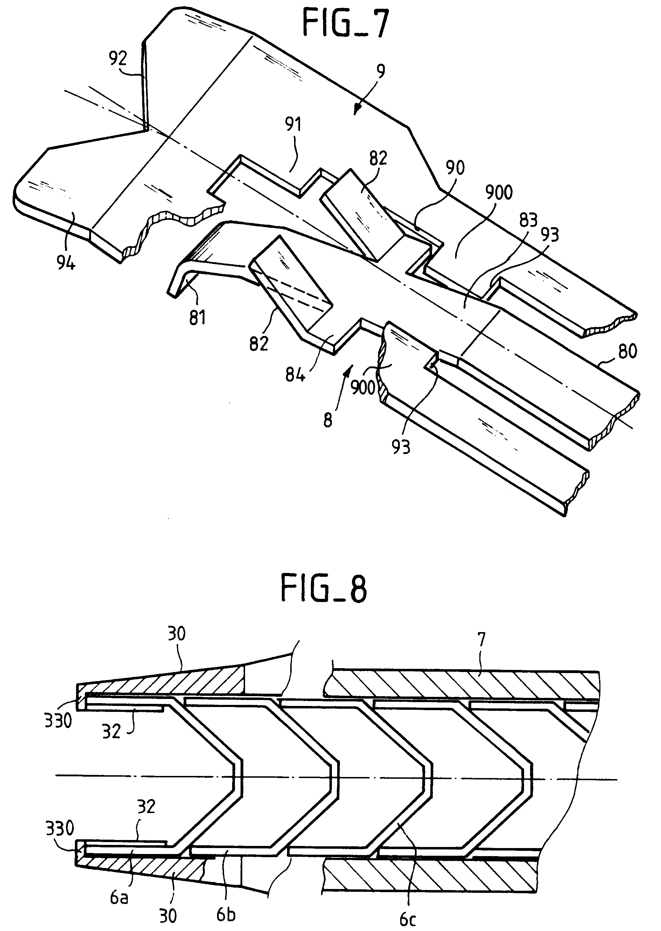

Comme on le voit plus particulièrement à la figure 7, la lame 8 possède une partie plate 80 par laquelle elle est soudée au coulisseau 2, et se raccorde à la languette 81 par l'intermédiaire d'une partie 83 inclinée vers le bas. Cette partie présente un élargissement 84 dans laquelle sont découpées deux languettes latérales 82 rabattues vers l'avant (côté languette 81) et vers le haut. L'ensemble de la languette 8 s'inscrit à l'intérieur d'une ouverture 90 formée dans une plaque plane 9 solidaire du corps fixe 1.

L'ouverture 90 a une forme générale rectangulaire, présentant dans sa partie centrale un rétrécissement formé de deux languettes rectangulaires 900 tournées vers l'intérieur de l'ouverture, et dont les bords arrière sont référencés 93. Ces derniers sont adaptés pour servir de contre-rampes aux pattes inclinées 82 lorsque le coulisseau 2 avance.

La zone avant 94 de la plaque 9 est légèrement inclinée vers le bas et son bord avant présente une échancrure en "V" 92, dont le rôle sera expliqué plus loin.

Selon une caractéristique importante de l'invention, la lame flexible 8 est précontrainte de telle sorte que la languette 81 tend constamment à s'abaisser, à l'intérieur du magasin 7, dans la position illustrée à la figure 6.

En position reculée du coulisseau 2, la zone élargie 84 se trouve située en arrière des languettes fixes 900, et la languette se trouve donc normalement abaissée.

Lorsque le coulisseau avance, les pattes 82 rencontrent les bords arrière 93 de l'ouverture, et par effet de rampe, la lame 8 se déploie vers le haut, provoquant la remontée (et l'escamotage) de la languette 81.

En position avancée - illustrée à la figure 7 - la zone élargie 84 a franchi les languettes fixes 900, et la languette 81 est à nouveau abaissée.

Lors du recul du coulisseau, la zone 90 va passer cette fois sous les languettes fixes 900 et la languette 81 va donc rester en position basse.

Les mâchoires 30 se trouvent juste à la sortie 72 du magasin 7. Le plan général des mâchoires à ce niveau est légèrement incliné vers l'avant et vers le bas d'un angle aigu u par rapport à l'horizontale (voir figure 6). Cet angle est de préférence égal à 15° environ.

Sur les parois internes des mâchoires 30 sont prévues des butées frontales 330 servant d'arrêt à l'extrémité des branches du clip. Des butées basses 32 servent à sa retenue entre les mâchoires. D'autres butée peuvent être prévues, notamment pour éviter l'échappement du clip vers le haut, afin d'assurer son positionnement correct et sa bonne retenue entre les mâchoires, même si la pince est soumise à des mouvements intempestifs en directions variées (notamment si elle est retournée).

En position de repos, la pince se trouve dans la position de la figure 1, les leviers 4 étant écartés l'un de l'autre. Des moyens élastiques non représentés tels qu'un ressort, agissant soit sur les leviers 4 soit sur le coulisseau 2, maintiennent normalement les leviers 4 écartés et par conséquent le coulisseau 2 en position avancée. Dans cette position avancée du coulisseau, la zone élargie 84 se trouve au-delà (en avant) des languettes fixes 900, languette 81 abaissée.

Le ressort 71 repousse la rangée de clips vers la sortie du magasin, et le premier clip, désigné 6a, se trouve en butée à l'intérieur des mâchoires 30 ; le second clip 6b est en appui contre le clip 6a, avec un décalage angulaire dû à l'inclinaision (d'angle u) des mâchoires par rapport au magasin. La languette 81 se trouve entre les branches du clip 6b.

Le clip 6a étant en place entre les mâchoires 30, la pince est utilisée de la manière suivante.

Le chirurgien positionne la pince de telle manière que le vaisseau à ligaturer VS s'insère entre les mâchoires. Cette opération est facilitée grâce à l'échancrure en "V" 92 qui assure le bon centrage du vaisseau dans la zone centrale des mâchoires. Le chirurgien actionne ensuite les deux leviers 4 à la manière d'une paire de ciseaux pour les refermer, ce qui a pour effet de faire reculer le coulisseau 2. La languette 81 vient en contact avec la portion transversale du clip 6b et fait reculer celui-ci, ainsi que toute la rangée de clips 6, à l'encontre du ressort de poussée 71 qui se comprime. Ceci a pour effet d'isoler du reste des clips le clip 6a situé entre les mâchoires. Dans le même mouvement de recul du coulisseau 2, comme déjà expliqué, les mâchoires 30 se resserrent (flèches F3, figure 10) réalisant l'écrasement du clip et donc la suture du vaisseau.

Une fois que cette opération a été effectuée, le chirurgien libère les leviers 4 qui s'écartent l'un de l'autre (flèches F'1 à la figure 2) ceci provoque l'avance du coulisseau 2 (flèche F'2, figures 2 et 11). Les mâchoires 30 peuvent alors s'ouvrir (flèches F'3, figure 11).

Les pattes 82 viennent dans le même temps en contact avec les bords 93 ce qui provoque la remontée et l'escamotage provisoire de la languette de retenue 81. Le clip 6b, toujours poussé par le ressort 71 vient alors se loger entre les mâchoires 30, remplaçant le clip 6a déjà posé. Le clip suivant 6c vient le remplacer à l'extrémité ouverte du magasin.

L'avance du coulisseau se poursuivant, une fois que la zone élargie 84 a franchi les languettes fixes 900, la lame flexible 8 se déploie alors vers le bas, et la languette de retenue 81 vient se positionner en position d'interception du clip 6c.

L'opération qui vient d'être décrite est réitérée ensuite autant de fois que nécessaire au cours de l'intervention chirurgicale, le cas échéant jusqu'à épuisement complet de la réserve contenue dans le magasin 7.

## Revendications

1. Pince chirurgicale pour poser des clips de suture en forme générale de "U", du type comprenant un corps fixe (1) sur lequel sont articulés deux leviers de manoeuvre (4), du genre branches de ciseaux, ce corps fixe (1) contenant un magasin linéaire (7) présentant une extrémité ouverte (72) et renfermant une réserve de clips (6) disposés à plat les uns contre les autres, leur ouverture tournée vers ladite extrémité ouverte (72), la pince comportant une paire de mâchoires (30) situées dans le prolongement du magasin (7), juste à la sortie de son extrémité ouverte (72), qui sont adaptées pour retenir et pour sertir l'un après l'autre des clips (6) provenant du magasin (7), caractérisée par le fait qu'elle comporte un coulisseau (2) disposé longitudinalement au-dessus du magasin (7) et dont le mouvement de translation longitudinal est commandé par lesdits leviers de manoeuvre (4), la fermeture (F1) de ceux-ci provoquant le déplacement du coulisseau dans le sens (F2) opposé à l'extrémité ouverte (72) - ou recul -, tandis que leur ouverture (F'1) provoque le déplacement du coulisseau en sens inverse (F'2) - ou avance -, qu'elle comporte également un ressort de poussée (71) qui tend constamment à déplacer la réserve de clips (6) vers l'extrémité ouverte (72) du magasin, et que ledit coulisseau (2) est solidaire, d'une part de moyens (20) assurant la fermeture des mâchoires (30) au cours de son recul (F2), d'autre part d'une languette de retenue (81) du premier clip (6b) contenu - côté extrémité ouverte (72) - dans le magasin (7), des moyens (82, 91) assurant l'escamotage momentané de cette languette (81) au cours de son avance (F'2).

2. Pince chirurgicale selon la revendication 1, caractérisée par le fait que lesdites mâchoires (30) sont plates et légèrement inclinées vers le bas par rapport au magasin (7).

3. Pince chirurgicale selon l'une des revendications 1 ou 2, caractérisée par le fait que lesdites mâchoires (30) sont les extrémités des branches (33) formées dans une même pièce (3) et séparées par une fente (34), la fermeture (F3) des mâchoires (30) se faisant sous déformation élastique de ces branches.

4. Pince chirurgicale selon la revendication 3, caractérisée par le fait que lesdits moyens (20) portés par le coulisseau (2) et qui assurent la fermeture des mâchoires (30) consistent en un étrier (200) en forme de "U" renversé et dont les branches latérales s'appliquent contre les surfaces de came (31) formées sur les bords extérieurs des branches (33) des mâchoires.

5. Pince chirurgicale selon l'une des revendications 1 à 4, caractérisée par le fait que lesdites mâchoires sont munies de butées d'arrêt et de positionnement (330, 32) des clips (6).

6. Pince chirurgicale selon l'une des revendications 1 à 5, caractérisée par le fait que ladite languette de retenue (81) est constituée par l'extrémité recourbée d'une lame flexible (8) fixée au coulisseau (2), et dont la précontrainte tend constamment à repousser la languette vers le bas, à l'intérieur du magasin (7), en position d'interception des clips (6).

7. Pince chirurgicale selon l'une des revendications 1 à 6, caractérisée par le fait que les moyens qui assurent l'escamotage momentané de la languette de retenue (81) comprennent une rampe inclinée (82) solidaire de la languette et une partie fixe (91) (solidaire du corps (1)) formant contre-rampe.

8. Pince chirurgicale selon l'une des revendications 6 et 7 prises en combinaison, caractérisée par le fait que ladite rampe inclinée (82) est formée de deux pattes découpées et repliées de la lame flexible (8), tandis que la partie fixe (91) formant contre-rampe est constituée par des languettes (900) formant les bords d'une ouverture (90) recevant la lame (8), et ménagée dans une plaque (9).

9. Pince chirurgicale selon la revendication 8, caractérisée par le fait que ladite plaque (9) possède un bord qui présente une échancrure en "V" (92) surplombant les mâchoires (30) et servant au centrage du vaisseau à suturer.

10. Pince chirurgicale selon l'une des revendications 1 à 9, caractérisée par le fait que la transmission du mouvement des leviers de manoeuvre (4) au coulisseau (2) est réalisée au moyen d'un jeu de biellettes (5).
